# EUROPEAN PATENT APPLICATION

(11) **EP 1 111 067 A2**
(43) Date of publication of application: **27.06.2001**
(21) Application number: 00126114.8
(22) Date of filing: 29.11.2000
(51) Int. Cl.: C12P 23/00, C12N 9/08, C12N 9/02

(54) **Recombinant production of carotenoids, particularly astaxanthin**

(30) Priority: 01.12.1999 EP 99123821
(71) Applicant: F. HOFFMANN-LA ROCHE AG, 4070 Basel (CH)
(72) Inventor: Hoshino, Tatsuo, Kamakura-shi, Kanagawa-ken 248-0027 (JP); Ojima, Kazuyuki, Fujisawa-shi, Kanagawa-ken 251-0877 (JP); Setoguchi, Yutaka, Fujisawa-shi, Kanagawa-ken 251-0033 (JP)
(74) Representative: Kellenberger, Marcus Dr.

(57) **Abstract**

The present invention is directed to a process for producing carotenoids comprising cultivating a recombinant organism having a gene for one or more active oxygen species-quenching factor(s) that is substantially disrupted with a disruption cassette specific to the gene, and recovering carotenoids from the culture, as well as to genetic materials useful in the said process, such as a recombinant organism producible of carotenoids, a disruption cassette, a recombinant DNA sequence, a recombinant DNA fragment; and a polynucleotide.

## Description

The present invention relates to recombinant production of carotenoids, particularly astaxanthin, and biological materials useful therefor.

Astaxanthin is known to be distributed in a wide variety of organisms such as animal (e.g. birds such as flamingo and scarlet ibis, and fish such as rainbow trout and salmon), algae and microorganisms. It is also recognized that astaxanthin has a strong antioxidation property against active oxygen as well as most of carotenoids, which is expected to apply to pharmaceutical usage to protect living cells against some diseases such as a cancer. Moreover, from a viewpoint of industrial application, a demand for astaxanthin as a coloring reagent is increasing especially in the industry of farmed fish, such as salmon, because astaxanthin imparts distinctive orange-red coloration to the animals and contributes to consumer appeal in the marketplace.

*Phaffia rhodozyma* is known as a carotenogenic yeast strain which produces astaxanthin specifically. Different from the other carotenogenic yeast, *Rhodotorula* species, *Phaffia rhodozyma* can ferment some sugars such as D-glucose. This is an important feature from a viewpoint of industrial application. In a recent taxonomic study, a sexual cycle of P. *rhodozyma* was revealed and its telemorphic state was designated under the name of *Xanthophyllomyces dendrorhous* (W.I. Golubev; *Yeast* 11, 101 - 110, 1995). Some strain improvement studies to obtain hyper producers of astaxanthin from *P. rhodozyma* have been conducted, but such efforts have been restricted to employ the method of conventional mutagenesis and protoplast fusion in this decade. Recently, Wery *et al.* developed a host vector system using *P. rhodozyma* in which a non-replicable plasmid was used to be integrated onto the genome of *P. rhodozyma* at the locus of ribosomal DNA in multicopies (Wery *et al., Gene,* 184, 89-97, 1997). And Verdoes *et al.* reported more improved vectors to obtain a transformant of *P. rhodozyma* as well as its three carotenogenic genes which code for the enzymes that catalyze the reactions from geranylgeranyl pyrophosphate to betacarotene (International patent WO97/23633). The importance of genetic engineering method on the strain improvement study of *P. rhodozyma* will increase in near future to break through the reached productivity by the conventional methods.

As described above, astaxanthin has an antioxidant property and this feature seems to have an important role *in vivo* for a protection against active oxygen species such as O₂., H₂O₂ and OH., which are continuously generated in living cells. An *et al.* obtained hyperproducer of astaxanthin from *P. rhodozyma* by selection of antimycin-sensitive strain after a conventional chemical mutagenesis (An, G-H. *et al., Appl. Env. Microbiol.,* 55 (1), 116-124, 1989). Antimycin is known to be an inhibitor of respiratory chain between cytochrome b and C₁ (Lucchini, G. *et al., Mol. Gen. Genet.,* 177, 139-, 1979) and enhanced pigmentation in such an antimycin-sensitive mutants. Active oxygen species produced due to a blockade of the primary respiratory chain at the bcₗ complex stimulated carotenoid formation (An, G-H *et al., Appl. Env. Microbiol.,* 55, 116-124, 1989). Indeed, addition of an O₂. generator, duroquinone, to the growth medium increased total carotenoid content (main carotenoid is astaxanthin) as well as the relative amounts of xanthophylls present in *P. rhodozyma,* while the active oxygen species-quenching factor mannitol reversed this effect (Schroeder, W. A. *et al., J. Gen. Microbiol.,* 139, 907-912, 1993). These results prompted such authors to speculate an antioxidant property of astaxanthin in *P. rhodozyma.* In fact, astaxanthin production is stimulated in post-exponential growth phase when respiration activity is fully induced. Moreover, the addition of respiratory substrate such as ethanol to the medium enhanced the astaxanthin production in *P. rhodozyma* (Gu, W-L. *et al·, J, Ind. Microbiol. Biotechnol.,* 19, 114-117, 1997). Although Schroeder *et al.* tried to determine a relationship of activities of superoxide dismutase (SOD) and catalase which act as native active oxygen species-quenching factors in *P. rhodozyma* to the productivity of astaxanthin by comparing a difference between parent strain and antimycin-sensitive hyperproducer of astaxanthin, direct correlation *of in vitro* activity could not be observed.

In accordance with the present invention the genes and the enzymes for active oxygen species-quenching factor(s), such as SOD and catalase which are biological materials useful in the improvement of the carotenoid, particularly astaxanthin, production process are provided. This invention involves cloning and determination of the genes which code for mitochondrial and cytoplasmic SODs and catalase. This invention also involves the enzymatic characterization as a result of the disruption of the genes in *P. rhodozyma.* Their disruption effects on the carotenogenesis can be confirmed by the cultivation of such transformants in an appropriate medium under an appropriate cultivation condition. More particularly, the present invention provides a process for producing carotenoids which comprises cultivating a recombinant organism whose gene for one or more active oxygen species-quenching factor(s) is substantially disrupted with the aid of a disruption cassette specific to the said gene, and recovering carotenoids from the culture. The host organism of the said recombinant organism may belong to the kingdom *of Monera*, *Protista or Fungi.* More preferable host organism of the said recombinant organism may belong to the genus *Erwinia*, *Rhodobacter*, *Myxococcus*, *Flavobacter*, *Paracoccus*, *Synechococcus*, *Synechocystis*, *Agrobacterium*, *Streptomyces*, *Haematococcus*, *Dunaliella*, *Phaffia*, *Xanthophyllomyces*, *Neurospora*, *Rhodotorula*, *Blakeslea*, or *Phycomyces*. Most preferably, the said host organism is a strain of *P. rhodozyma*

The active oxygen species-quenching factor(s) is(are) mitochondrial superoxide dismutase (SOD), cytoplasmic superoxide dismutase (SOD) and/or catalase.

The present invention also provides a recombinant organism producible of carotenoids, which is characterized in that the gene for at least one active oxygen species-quenching factor is substantially disrupted with the aid of introduction of a disruption cassette specific to the said gene. The active oxygen species-quenching factor(s) to be disrupted is(are) mitochondrial superoxide dismutase (SOD), cytoplasmic superoxide dismutase (SOD) and/or catalase.

The present invention further provides a disruption cassette which can be used to prepare the said recombinant organism of the present invention for disrupting a gene coding for an active oxygen species-quenching factor effective in an organism which is producible of carotenoids, which comprises a partial nucleotide sequence substantially identical to a part of the DNA sequence coding for an active oxygen species-quenching factor and a selectable marker gene. For the construction of the disruption cassette, the target organism may belong to the kingdom of *Monera*, *Protista or Fungi*, more preferably to the genus *Erwinia*, *Rhodobacter*, *Myxococcus*, *Flavobacter*, *Paracoccus*, *Synechococcus*, *Synechocystis*, *Agrobacterium*, *Streptomyces*, *Haematococcus*, *Dunaliella*, *Phaffia, Wanthophyllomyces*, *Neurospora*, *Rhodotorula*, *Blakeslea*, or *Phycomyces*. The active oxygen species-quenching factor(s) to be disrupted is(are) mitochondrial superoxide dismutase (SOD), cytoplasmic superoxide dismutase (SOD) and/or catalase.

As used herein, a polynucleotide or polypeptide sequence (A) is said to be substantially identical to another sequence (B) if sequence A is at least 75% identical, preferably 85% identical, such as at least 95% identical to sequence B.

Further, the present invention provides a recombinant DNA sequence coding for an active oxygen species-quenching factor effective in an organism producible of carotenoids. The DNA sequence may be originated from an organism belonging to the kingdom of *Monera*, *Protista or Fungi,* more preferably to the genus *Erwinia, Rhodobacter, Myxococcus, Flavobacter*, *Paracoccus*, *Synechococcus*, *Synechocystis, Agrobacterium*, *Streptomyces*, *Haematococcus*, *Dunaliella*, *Phaffia*, *Xanthophyllomyces*, *Neurospora*, *Rhodotorula*, *Blakeslea*, or *Phycomyces*. Particularly preferred organism is *P. rhodozyma.* The active oxygen species-quenching factor coded by the recombinant DNA sequence may be mitochondrial superoxide dismutase, cytoplasmic superoxide dismutase and/or catalase.

A recombinant DNA sequence coding for mitochondrial superoxide dismutase can be identified by SEQ ID NO: 1 or 4, or it may have high homology to the sequence of SEQ ID NO: 1 or 4 enough to recombine with either of the sequences of SEQ ID NOs: 1 and 4. A recombinant DNA sequence coding for cytoplasmic superoxide dismutase can be identified by SEQ ID NO: 2 or 6, or it may have high homology to the sequence of SEQ ID NO: 2 or 6 enough to recombine with either of the sequences of SEQ ID NOs: 2 and 6. Also a recombinant DNA sequence coding for catalase can be identified by SEQ ID NO: 3 or 8, or it may have high homology to the sequence of SEQ ID NO: 3 or 8 enough to recombine with either of the sequences of SEQ ID NOs: 3 and 8.

In the present invention, any combination of the following hybridization and wash conditions may be used, as appropriate, to identify homologous polynucleotide sequences:

### High Stringency Hybridization:

6X SSC
0.5% SDS
100 ug/ml denatured salmon sperm DNA
50% formamide
Incubate overnight with gentle rocking at 42°C overnight.

### High Stringency Wash:

1 wash in 2X SSC, 0.5% SDS at Room Temperature for 15 minutes, followed by another wash in 0.lx SSC, 0.5% SDS at Room Temperature for 15 minutes.

### Low Stringency Hybridization:

6X SSC
0.5% SDS
100 ug/ml denatured salmon sperm DNA
50% formamide
Incubate overnight with gentle rocking at 37°C overnight.

### Low Stringency Wash:

1 wash in 0.1X SSC, 0.5% SDS at Room Temperature for 15 minutes.

Moderately stringent conditions may be obtained by varying the temperature at which the hybridization reaction occurs and/or the wash conditions as set forth above.

Thus, as used herein, a sequence (A) is said to have "high homology" to another sequence (B) if sequence A hybridizes to sequence B under high stringency conditions *(i.e.,* high stringency hybridization and wash conditions as defined above), and if the polypeptide or polypeptide fragment encoded by sequence A has the same activity as the polypeptide encoded by B.

The present invention further provides a recombinant DNA fragment which comprises a coding region for a transit peptide upstream the coding region of an objective protein which may be mitochondrial superoxide dismutase. The expression of this recombinant DNA fragment enables to locate the objective protein in mitochondria. Thus the present invention also provides a method for locating an objective protein in mitochondria which comprises expressing the recombinant DNA fragment comprising a coding region for a transit peptide upstream the coding region of an objective protein in an appropriate recombinant host organism.

As mentioned above, the present invention discloses the nucleotide sequences of active oxygen species-quenching factors, such as mitochondrial superoxide dismutase, cytoplasmic superoxide dismutase and catalase. These polynucleotides are provided for use as probe or primer for cloning gene for the active oxygen species-quenching factor(s) effective in another organism producible of carotenoids on the basis of the homology of the genes.

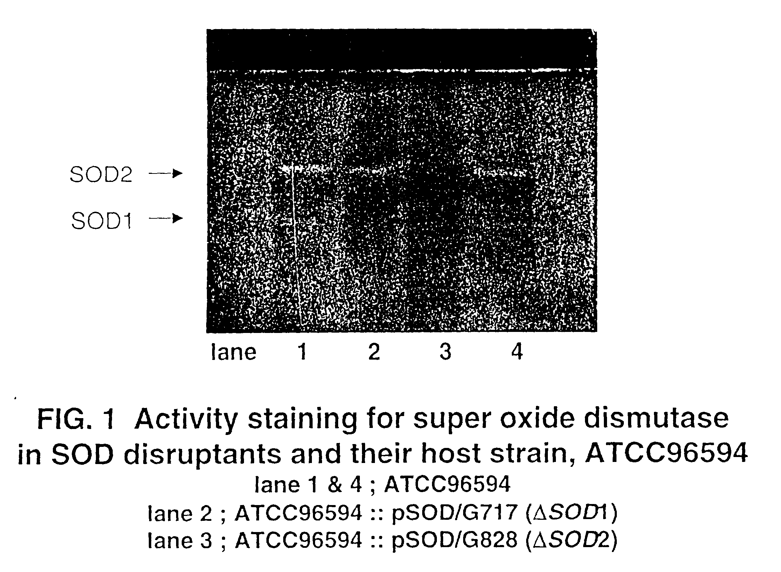

FIG. 1 shows the activity staining for superoxide dismutase after native polyacrylamide gel electrophoresis by using cell-free extracts obtained from ATCC 96594 and its *SOD* mutants. Lane 1, *P. rhodozyma* ATCC 96594; Lane 2 *P. rhodozyma* ATCC 96594 :: pSOD/G717 (SOD1 disruptant); Lane 3 *P*. *rhodozyma* ATCC 96594 :: pSOD/G828 (SOD2 disruptant); Lane 4 *P. rhodozyma* ATCC 96594

As it is briefly explained above, the object of the present invention is to provide a novel process for producing carotenoids biologically. The novel process comprises cultivating a recombinant organism whose gene for one or more active oxygen species-quenching factor(s) is substantially disrupted with the aid of a disruption cassette specific to the said gene, and recovering carotenoids from the culture.

For the purpose of the present invention, there is also provided a recombinant DNA sequence which contains an open reading frame coding for active oxygen species-quenching factor(s), which may be an enzyme, such as mitochondrial SOD or cytoplasmic SOD. The recombinant DNA sequence may contain a partial fragment encoding catalase gene. These sequences are useful to construct the said disruption cassette, as they are able to recombine with the native gene(s) for the said enzymes so as to specifically disrupt the gene(s).

The said recombinant DNA sequence may be the one which can be derived from an organism belonging to the kingdom of *Monera*, *Protista or Fungi,* more preferably to the genus *Erwinia*, *Rhodobacter*, *Myxococcus*, *Flavobacter*, *Paracoccus*, *Synechococcus*, *Synechocystis*, *Agrobacterium*, *Streptomyces, Haematococcus*, *Dunaliella*, *Phaffia*, *Xanthophyllomyces*, *Neurospora*, *Rhodotorula*, *Blakeslea,* or *Phycomyces*. Particularly preferred organism is *P*. *rhodozyma*. The active oxygen species-quenching factor coded by the recombinant DNA sequence may be mitochondrial SOD, cytoplasmic SOD and/or catalase. A specific example of the recombinant DNA sequence is derived from a gene of *Phaffia rhodozyma* and is selected from (i) a DNA sequence represented in SEQ ID NO: 1 or 2; (ii) those cDNAs identified by SEQ ID NO: 4 or 6; (iii) an isocoding or an allelic variant for the DNA sequence represented in SEQ ID NO: 1, 2, 4 or 6; and (iv) a derivative of a DNA sequence represented in SEQ ID NO: 1, 2, 4 or 6 with addition, insertion, deletion and/or substitution of one or more nucleotide(s), and coding for a polypeptide having the said enzyme activity. The said recombinant DNA sequence may also be characterized in that (a) it codes for the said enzyme having an amino acid sequence selected from the group consisting of those described in SEQ ID NOs: 5 and 7, or (b) it codes for a variant of the said enzyme selected from (i) an allelic variant, and (ii) an enzyme having one or more amino acid addition, insertion, deletion and/or substitution and having the stated enzyme activity.

As used herein, an "allelic variant" means the variant that has at least one mutation in either one of two alleles in the diploid organism such as *Phaffia rhotlozyma*, *Xanthophyllomyces dendrorhous* and the like. Both alleles of a given gene are concerned with the same trait or characteristic, but the product or function coded for by a particular allele differs, qualitatively and/or quantitatively, from that coded for by other alleles of that gene.

Allelic variant can occur naturally or be generated artificially by means of chemical mutagenesis. A wild type allele is one which codes for a particular phenotypic characteristic found in the wild type strain of a given organism.

As used herein, an "isocoding variant" means the variant in which the nucleotide sequence of a given gene differs from the sequence from the wild type gene although its translated product (i.e. amino acid sequence) is identical with that from the wild type protein. This is caused by degeneracy of genetic code and by the difference of codon usage which is not identical among various organisms.

As used herein, a "derivative of a DNA sequence" is a DNA sequence that encodes a polypeptide having the activity of the corresponding SEQ ID NO but which differs from that DNA sequence by from 1-20, preferably 1-10, such as 1-5 nucleotide additions, insertions, deletions, and/or substitutions.

Particularly specified recombinant DNA sequence mentioned above may be that which can be derived from a gene of *Phaffi*a *rhodozyma* and is selected from (i) a DNA sequence represented in SEQ ID NO: 3; (ii) cDNA identified by SEQ ID NO: 8; (iii) an isocoding or an allelic variant for the DNA sequence represented in SEQ ID NO: 3 or 8; and (iv) a derivative of a DNA sequence represented in SEQ ID NO: 3 or 8 with addition, insertion, deletion and/or substitution of one or more nucleotide(s), and coding for a polypeptide having the said enzyme activity. The said recombinant DNA sequence may also be characterized in that (a) it codes for the said enzyme having a partial amino acid sequence selected from the group consisting of those described in SEQ ID NO: 9, or (b) it codes for a variant of the said enzyme selected from (i) an allelic variant, and (ii) an enzyme having one or more amino acid addition, insertion, deletion and/or substitution and having the stated enzyme activity. Such a recombinant DNA sequence may preferably be in the form of a vector.

The present invention also provides the use of the said recombinant DNA sequence to transform a host organism so as to obtain an organism whose gene for at least one active oxygen species-quenching factor is substantially disrupted with the aid of introduction of the said disruption cassette specific to the said gene.

As used herein, a gene is "disrupted" or "substantially disrupted" if the activity of the polypeptide that it encodes is reduced relative to a non-disrupted gene. Preferably, the activity is reduced by 10%, preferably by at least 50% such as for example by at least 75%, more preferably by at least 90% to 100%.

A convenient form of the recombinant DNA sequence may be a vector. The recombinant organism obtained by use of the recombinant DNA is disrupted in its DNA sequence encoding mitochondrial SOD, cytoplasmic SOD, or catalase. The host organism transformed with the recombinant DNA is useful in the improvement of the production process of carotenoids, in particular astaxanthin. Thus the present invention also provides such a recombinant organism.

This biological production method of carotenoids can improve the productivity of carotenoids, in particular of astaxanthin. Thus the method for producing a carotenoid, characterized in that a recombinant organism described above is cultivated under conditions conductive to the production of the carotenoid is one of the aspect of the present invention. This method may be applied to the biological production of astaxanthin.

Many researchers pointed out that active oxygen species might stimulate carotenoid production in known carotenogenic organisms. Carotenoid biosynthesis in cyst cells of *Haematococcus pluvialis* is enhanced by environmental oxidative stress (Kobayashi et al., *Appl. Env*, *Microbiol.,* 59, 867-873, 1993). Carotenoid biosynthesis might be induced by active oxygen species and the accumulated carotenoids might function as a protective agent against oxidative stress damage in *Dunuliella bardawil* (Shaish et al., *Planta*, 190, 363-368, 1993). Although astaxanthin production in *P. rhodozyma* was studied *in vivo* under various cultivation conditions in which a generation of active oxygen species was altered, a correlation between active oxygen generated and carotenoids productivity was not clearly determined, probably because native active oxygen species-quenching factors were still present in such experiments and rescued the effects of active oxygen species on the carotenoids production to some extent (Schroeder, W. A. *et al*., *J*. *Gen*. *Microbiol*., 139, 907-912,1993).

In this invention, to exclude the possibility that existence of native active oxygen species-quenching factor in *P. rhodozyma* can quench the positive effect by active oxygen on astaxanthin production, such native active oxygen species-quenching factors as SOD and catalase were cloned from *P*. *rhodozyma* to disrupt their expression by constructing and introducing gene disruption plasmids. On the assumption that astaxanthin would play an antioxidant role in *P. rhodozyma*, inactivation of native active oxygen species-quenching factor may affect to carotenoids production probably because relatively increased active oxygen species *in vivo* due to absence of native active oxygen species-quenching factors would stimulate a production of astaxanthin as an alternative player to quench active oxygen species.

Active oxygen species have toxicity to living cells through the oxidative damage to the intercellular molecules such as proteins or nucleic acids. Recent studies revealed that aging is caused by oxidative damage by demonstrating a correlation between increased superoxide dismutase activity, increased life-span, and decreased oxidative damage in fruit flies and nematodes (Agarwal, S. et al., *Proc. Natl. Acad*. *Sci. U. S. A.,* 91, 12332-12335, 1994, Larsen, P. L. *Proc. Natl. Acad*. *Sci. U. S. A.,* 90, 8905-8909, 1993, Sohal, R. S. et al., *J. Biol. Chem*., 270, 15671-15674, 1995). SOD and related antioxidant enzymes and their genes are studied well in procaryote and eukaryote.

Yeast, *S. cerevisiae*, like most eucaryotes, contains Cu/ZnSOD (product of the *SOD1* gene) in the cytosol and MnSOD (product of the *SOD2* gene) in the mitochondria. These enzymes catalyze the disproportionating of O₂., producing O₂ and H₂O₂. Together with small molecular antioxidants, such as glutathione and ascorbate; other antioxidant enzymes, such as catalases and peroxidases; and metal chelating proteins such as metallothionein, they allow aerobes to survive under O₂, presumably by minimizing oxidative damage. The importance of cytoplasmic SOD was demonstrated by the high sensitivity to dioxygen shown by *S. cerevisiae* and *Escherichia coli* devoid of *SOD.* In both organisms, the loss of SOD activity is associated with slow growth in aerobic conditions, with higher mutation rates and with specific biosynthetic defects. *(sod1*^{*-*} yeast requires lysine and methionine for aerobic growth, whereas *sod- E. coli* requires branched amino acids). In some cases these effects are known to be due to the inhibitory effect of superoxide on iron sulfur cluster proteins (Gardner, P. R. *et al*., *J*. *Biol*. *Chem*., 266, 19328-19333, .1991, Kuo, C. F., et *al*., *J*. *Biol*. *Chem*., 262, 4724-4727, 1987). Mutants *of sod2* of *S. cerevisiae* are little affected when grown in air with glucose as the carbon source. However, they are highly sensitive to hyperoxia and grow poorly in nomoxia in carbon sources that require respiration for their metabolism.

Because genes coding for SOD and catalase have been cloned from other species, corresponding genes from *P. rhodozyma* could be cloned with degenerate PCR method. At first, we cloned a partial gene fragment containing a portion of *SOD* gene and *CAT* gene by using above method. The said degenerate PCR is a method to clone a gene of interest which has high homology of amino acid sequence to the known enzyme from other species which has a same or similar function. Degenerate primer, which is used as a primer in degenerate PCR, was designed by a reverse translation of the amino acid sequence to corresponding nucleotides ("degenerated"). In such a degenerate primer, a mixed primer which consists any of A, C, G or T, or a primer containing inosine at an ambiguity code is generally used. In this invention, such the mixed primers were used for degenerate primers to clone above genes. PCR condition used is varied depending on primers and genes to clone as described hereinafter. In this invention, two species of *SOD* genes whose sequence was different from each other were cloned from the same PCR band in degenerate PCR and named as *SOD1* and *SOD2* genes.

An entire gene containing its coding region with its intron as well as its regulation region such as a promoter or terminator can be cloned from a chromosome by screening of genomic library which is constructed in phage vector or plasmid vector in an appropriate host, by using a partial DNA fragment obtained by degenerate PCR as described above as a probe after it was labeled. Generally, *E. coli* as a host strain and *E*. *coli vector*, a phage vector such as λ phage vector, or a plasmid vector such as pUC vector are often used in the construction of library and a following genetic manipulation such as a sequencing, a restriction digestion, a ligation and the like. In this invention, an *EcoR* Igenomic library of *P. rhodozyma* was constructed in the derivatives of λ vector, λZAPII and λDASHII depending on an insert size. An insert size, what length of insert must be cloned, was determined by the Southern blot hybridization for each gene before a construction of a library. In this invention, a DNA which was used for a probe was labeled with digoxigenin (DIG), a steroid hapten instead of conventional ³²P label, following the protocol which was prepared by the supplier (Boehringer-Mannheim (Mannheim, Germany)). A genomic library constructed from the chromosome of *P. rhodozyma* was screened by using a DIGlabeled DNA fragment which had a portion of a gene of interest as a probe. Hybridized plaques were picked up and used for further study. In the case of using λDASHII (insert size was from 9 kb to 23 kb), prepared λDNA was digested by the *EcoR* I, followed by the cloning of the *EcoR*I insert into a plasmid vector such as pUC19 or pBluescriptII SK+. When AZAPII was used in the construction of the genomic library, *in vivo* excision protocol was conveniently used for the succeeding step of the cloning into the plasmid vector by using a derivative of single stranded M13 phage, Ex assist phage (Stratagene, La Jolla, USA). A plasmid DNA thus obtained was examined for sequencing. In this invention, *SOD1* and *SOD2* genes were obtained from the λZAPII library independently from each other and catalase *(CAT)* gene was cloned from λDASHII library.

In this invention, we used the automated fluorescent DNA sequencer, ALFred system (Pharmacia, Uppsala, Sweden) using an autocycle sequencing protocol in which the Taq DNA polymerase is employed in most cases of sequencing.

In this invention, the inventors determined the genomic sequence containing an open reading frame of *SOD1* gene or *SOD2* gene as well as its promoter and terminator sequence. From sequence analysis, it was found that *SOD1* codes for mitochondrial SOD, judged from a presence of transit peptide at its amino terminal end. On the contrary, *SOD2* does not have such a transit peptide sequence and this fact suggested that *SOD2* codes for cytoplasmic SOD. The inventors also determined a partial genomic sequence of an open reading frame for *CAT* gene.

Transit peptide is a signal sequence to transfer the nucleic gene products, which code on chromosome, and whose translated proteins function in mitochondria, to mitochondrial location of proteins, such as enzymes involved in TCA cycle. To express some proteins in mitochondria, addition of transit peptide at its amino terminal end is useful.

In this invention, disruption plasmids for *SOD1*, *SOD2* and *CAT* genes were constructed by ligating partial fragments of above genes, which do not contain the either end of genes, with drug resistant genes to suicidal vector, which cannot be autonomously replicated in *P. rhodozyma* due to lack of autonomous replication sequence. A drug resistant gene which encodes the enzyme that enables the host survive in the presence of a toxic antibiotic is often used for the selectable marker. G418 resistance gene harbored in pPR2T (Verdoes et *al*. (International patent publication, WO97/23633)) is an example of a drug resistance gene. Such a suicidal vector cannot replicate by itself and can be present only in an integrated form on the chromosome of the host as a result of a single-crossing recombination using the homologous sequence between a vector and a chromosome. In the case of recombination with a gene of interest, its genetic sequence cannot be reconstituted on the chromosome of the host strain due to lack of either end of the gene and as a consequence, the gene of interest could be disrupted in the recombinant strain thus obtained. Another example for disruption plasmid is a double crossing over type of plasmid. This type of disruption plasmid contains two different partial fragments of objective gene to be disrupted and selective marker gene such as a drug resistant gene is inserted between such two fragments. After the recombination between the chromosome of the recipient cells and donor plasmid DNA at the two homologous parts of the gene, replacement of the chromosome sequence with the donor DNA occur and selective marker gene is inserted into the objective gene to be disrupted. In general, double crossing over type of plasmid has lower frequency of recombination than single crossing type of vector.

In this invention, inventors inactivated the enzymes of interest by their disruption of corresponding genes. The other way to evaluate an effect of gene product of an interest is to decrease its expression with genetic engineering methods. For this purpose, some methods were exploited. One of such methods is anti-sense method. Anti-sense method is a method to decrease an expression of gene of interest by introducing an artificial gene fragment, whose sequence is complementary to that of gene of interest. And such an anti-sense gene fragment form complex with mature mRNA fragment of objective gene *in vivo* and inhibit an efficient translation from mRNA, as a consequence.

The other method is a mutation of promoter region. In general, the gene consists of several parts which have different functions from each other. In eukaryotes, genes which encode corresponding protein are transcribed to premature messenger RNA (pre-mRNA) differing from the genes for ribosomal RNA (rRNA), small nuclear RNA (snRNA) and transfer RNA (tRNA). Although RNA polymerase II (PolII) plays a central role in this transcription event, PolII can not solely start transcription without *cis* element covering an upstream region containing a promoter and an upstream activation sequence (UAS), and a *trans*-acting protein factor. At first, a transcription initiation complex which consists of several basic protein components recognize the promoter sequence in the 5'-adjacent region of the gene to be expressed. In this event, some additional participants are required in the case of the gene which is expressed under some specific regulation, such as a heat shock response, or adaptation to a nutrition starvation, and so on. In such a case, a UAS is required to exist in the 5'-untranslated upstream region around the promoter sequence, and some positive or negative regulator proteins recognize and bind to the UAS. The strength of the binding of transcription initiation complex to the promoter sequence is affected by such a binding of the *trans*-acting factor around the promoter, and this enables the regulation of the transcription activity.

After the activation of a transcription initiation complex by the phosphorylation, a transcription initiation complex initiates transcription from the transcription start site. Some parts of the transcription initiation complex are detached as an elongation complex from the promoter region to the 3' direction of the gene (this step is called as a promoter clearance event) and an elongation complex continues the transcription until it reaches to a termination sequence that is located in the 3'-adjacent downstream region of the gene.

To decrease an expression of gene of interest, mutation by conventional chemical mutagenesis or genetic site-directed mutagenesis in the promoter region of objective gene containing UAS sequence described above was often used. Mutant strain in which expression of enzyme of interest might decrease can be obtained by transforming host strain with a recombinant DNA having such a mutated promoter region. As described above, such an attempt to decrease the expression of gene is also employed as well as gene disruption for objective to determine an effect of gene product on phenomena in living organisms.

As a transformation method, LiCI method (Wery *et al*., *Yeast*, 12 (7), 641-651, 1996) and electroporation method (Wery *et al*., *Gene*, 184, 89-97, 1997) were applied to transform *P. rhodozyma* but the efficiency of transformation under these conditions seemed to be still low. In this invention, Biolistic transformation method (Johnston *et al*., *Methods in Molecular Biology,* 53; 147-153, 1996) was applied for transformation of *P. rhodozyma*. Biolistic method is a simple and reliable protocol, in which donor DNA coated on gold or tungsten particle is shot into the living cells directly with high-pressured helium gas. This transformation protocol was successfully applied to *Cryptococcus neoformans* which belongs to basisdiomycetous yeast as well as *P. rhodozyma* and was difficult to transform with conventional transformation method (Toffaletti, *et al*., *J*. *Bacteriol*., 175 (5), 1405-1411, 1993). In this invention, this biolistic method was successfully used to transform *P. rhodozyma* cells.

The event of gene disruption can be confirmed by enzymatic characterization directly and by genetic analysis with PCR or Southern blot hybridization by using the chromosome obtained from transformants thus obtained. In this invention, a direct confirmation of SOD disruption were performed by activity staining and characterization of catalase disruption were conducted by visual observation like catalase test which is often used in bacterial taxonomy.

Such a genetically engineered *P. rhodozyma* would be cultivated in an appropriate medium and evaluated in its productivity of astaxanthin.

The present invention is further illustrated with Examples described below by referring to the attached drawings.

### Example

The following materials and methods were employed in the Example described below:

### Strain

*P. rhodozyma* ATCC 96594 (re-deposited under the accession No. ATCC 74438 on April 8, 1998 pursuant to the Budapest Treaty)
*E*. *coli* DH5a: F^{⁻}, φ80d, *lac*ZΔM15, Δ(*lac*ZYA-*arg*F)U169, *hsd* (r_{K}^{⁻}, m_{K}⁺), *rec*Al,*end* Al, *deo*R, *thi-*l, *sup*E44, *gyr*A96, *rel*A1 (Toyobo, Osaka, Japan)
*E. coli* XL 1 -Blue MRF': Δ(*mcr*A)183, Δ(*mcr*CB-*hsdSMR*-mrr)173, *end*A1, *sup*E44, *thi-1, rec*A1, *gyr*A96, *rel*A1, *lac*[F' *pro*AB,*lac*1 ^{q}*Z*ΔM15, Tn10 (tet^{^{r}})] (Stratagene, La Jolla, USA)
*E. coli* SOLR: *e14*^{^{*-*}}*(mcr*A), Δ(*mcr*CB-*hsd*SMR-mrr)171, *sbc*C, *rec*B, *rec*J, *umu*C :: Tn5(kan^{r}), *uvr*C, *lac*, *gyr*A96, *rel*A1, *thi-* 1, *end*A1, λ^{R}*,* [F*' pro*AB, *lac*l^{q}ZΔM15] Su^{⁻} (nonsuppressing) (Stratagene)
*E*. *coli* XL,*l* MRA (P2): *Δ*(*mcr*)A*183*, Δ(*mcr*CB-*hsd*SMR-*mrr*)173, *end*A1, *sup*E44, *thi-*1, *gyr*A96, *rel*A1, *lac* (P2 lysogen) (Staratgene)
*E*. *coli* TOP10: *F*^{*-*}, *mcr*A, Δ(*mrr*-*hsd*RMS-*mcr*)BC, φ*80*, *Δlac*Z M*15*, Δ*lac*X74, *rec*A1, *deo*R, *ara*D139, *(ara-leu)*7697, *gal*U, *gal*K, *rps*L (*Str*^{*r*}), *end*A1, *nup*G (Invitrogen, Carlsbad, USA)

### Vector

λZAPII (Stratagene)
λDASHII (Stratagene)
pBluescriptII SK+(Stratagene)
pCR2.1TOPO (Invitrogen)
pUC19 (Takara Shuzo, Otsu, Japan)

### Media

*P. rhodozyma* strain is maintained routinely on agar plate of YPD medium (DIFCO, Detroit, USA). *E. coli* strain is maintained in LB medium (10 g Bacto-trypton, 5 g yeast extract (DIFCO) and 5 g NaCI per liter). NZY medium (5 g NaCl, 2 g MgSO₄-7H₂O, 5 g yeast extract (DIFCO), 10 g NZ amine type A (WAKO, Osaka, Japan) per liter is used for λ phage propagation in a soft agar (0.7 % agar (WAKO)). When an agar medium was prepared, 1.5 % of agar (WAKO) was supplemented.

### Methods

A general method for techniques of molecular genetics was according to the method in Molecular cloning: a Laboratory Manual, 2nd Edition (Cold Spring Harbor Laboratory Press, 1989). Restriction enzymes and T4 DNA ligase were purchased from Takara Shuzo (Japan).

Isolation of a chromosomal DNA from *P. rhodozyma* was performed by using QIAGEN Genomic Kit (QIAGEN, Hilden, Germany) following the protocol supplied by the manufacturer. Mini-prep of plasmid DNA from transformed *E*. *coli* was performed with the Automatic DNA isolation system (Pl-50, Kurabo, Co. Ltd., Osaka, Japan). Midi-prep of plasmid DNA from an *E*. *coli* transformant was performed by using QIAGEN column (QIAGEN). Isolation of λ DNA was performed by Wizard lambda preps DNA purification system (Promega, Madison, USA) following the protocol prepared by the manufacturer. A DNA fragment was isolated and purified from agarose by using QIAquick or QIAEX II (QIAGEN). Manipulation of λ phage derivatives was followed by the protocol prepared by the manufacturer (Stratagene).

Isolation of total RNA from *P. rhodozyma* was performed with phenol method by using Isogen (Nippon Gene, Toyama, Japan). mRNA was purified from total RNA thus obtained by using mRNA separation kit (Clontech, Palo Alto, USA). cDNA was synthesized by using CapFinder cDNA construction kit (Clontech).

*In vitro* packaging was performed by using Gigapack III gold packaging extract (Stratagene).

Polymerase chain reaction (PCR) is performed with the thermal cycler from Perkin Elmer model 2400. Each PCR condition is described in examples. PCR primers were purchased from a commercial supplier or synthesized with a DNA synthesizer (model 392, Perkin Elmer, Japan, Urayasu, Japan). Fluorescent DNA primers for DNA sequencing were purchased from Pharmacia. DNA sequencing was performed with the automated fluorescent DNA sequencer (ALFred, Pharmacia).

Competent cells of *E*. *coli* DH5α were purchased from Toyobo (Japan).

The apparatus and reagent for biolistic transformation of *P. rhodozyma* were purchased from Nippon Bio-Rad Laboratories (Tokyo, Japan).

### Example 1: Isolation of mRNA from P. rhodozyma and construction of cDNA library

To construct cDNA library of *P. rhodozyma,* total RNA was isolated by phenol extraction method right after the cell disruption and the mRNA from *P. rhodozyma* ATCC96594 strain was purified by using mRNA separation kit (Clontech).

At first, Cells of ATCC96594 strain from 10 ml of two-day-culture in YPD medium were harvested by centrifugation (1500 x g for 10 min.) and washed once with extraction buffer (10 mM Na-citrate / HCl (pH 6.2) containing 0.7 M KCI). After suspending in 2.5 ml of extraction buffer, the cells were disrupted by French press homogenizer (Ohtake Works Corp., Tokyo, Japan) at 1500 kgf/cm² and immediately mixed with two times of volume of isogen (Nippon gene) according to the method specified by the manufacturer. In this step, 400 µg of total RNA was recovered.

Then this total RNA was purified by using mRNA separation kit (Clontech) according to the method specified by the manufacturer. Finally, 16 µg of mRNA from *P. rhodozyma* ATCC 96594 strain was obtained.

To obtain cDNA species from *P.rhodozyma*, CapFinder PCR cDNA construction kit (Clontech) was used according to the method specified by the manufacturer. One µg of purified mRNA was applied for a first strand synthesis followed by PCR amplification. After this amplification by PCR, 1 mg of cDNA pool was obtained.

### Example 2: Cloning of two species of partial SOD gene from P. rhodozyma

To clone a partial *SOD* gene from *P*. *rhodozyma*, a degenerate PCR method was exploited. Two mixed primers whose nucleotide sequences were designed and synthesized as shown in TABLE 1 based on the common sequence of known superoxide dismutase genes from other species.

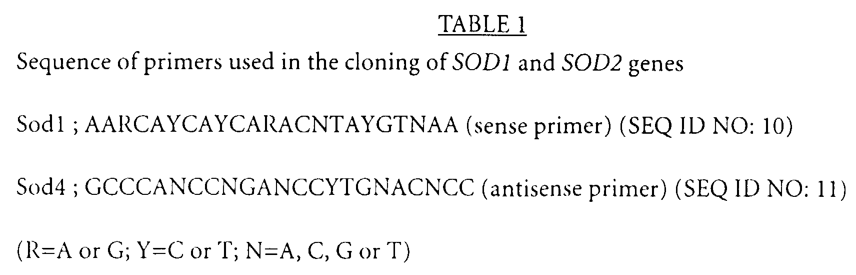

After the PCR reaction of 25 cycles of 94 °C for 15 seconds, 46 °C for 30 seconds and 72°C for 15 seconds by using ExTaq (Takara Shuzo) as a DNA polymerase and cDNA pool obtained in example 1 as a template, reaction mixture was applied to agarose gel electrophoresis. A PCR band that has a desired length was recovered and purified by QIAquick (QIAGEN) according to the method by the manufacturer and then ligated to pCR2.1-TOPO (Invitrogen). After the transformation of competent *E. coli* TOP10, 6 white colonies were selected and plasmids were isolated with Automatic DNA isolation system (Kurabo PI-50). As a result of sequencing, it was found that two clones had different sequences each other, whose deduced amino acid sequences were independently similar to known *SOD* genes. These isolated cDNA clones were designated as pSOD614 #2 and pSOD614 #3 and used for further study.

### Example 3: Isolation of genomic DNA from P. rhodozyma

To isolate a genomic DNA from *P. rhodozyma*, QIAGEN genomic kit was used according to the method specified by the manufacturer.

At first, cells of *P*. *rhodozyma* ATCC 96594 strain from 100 ml of overnight culture in YPD medium were harvested by centrifugation (1500 x g for 10 min.) and washed once with TE buffer (10 mM Tris / HCl (pH 8.0) containing 1 mM EDTA). After suspending in 8 ml of Y1 buffer of the QIAGEN genomic kit, lyticase (SIGMA) was added at the concentration of 2 mg/ml to disrupt cells by enzymatic degradation and the reaction mixture was incubated for 90 minutes at 30 °C and then proceeded to the next extraction step. Finally, 20 µg of genomic DNA was obtained.

### Example 4: Southern blot hybridization by using pSOD614 #2 and pSOD614 #3 as probes

Southern blot hybridization was performed to clone genomic fragment which contains *SOD* genes from *P. rhodozyma*. Two µg of genomic DNA was digested by *EcoR*I and subjected to agarose gel electrophoresis followed by acidic and alkaline treatment. The denatured DNA was transferred to nylon membrane (Hybond N+, Amersham) by using transblot (Joto Rika, Tokyo, Japan) for an hour. The DNA was transferred to nylon membrane and fixed by a heat treatment (80 °C, 90 minutes). Probes were prepared by labeling the template DNAs *(EcoR*l- digested pSOD614 #2 and pSOD614 #3) with DIG multipriming method (Boehringer Mannheim). Hybridization was performed with the method specified by the manufacturer. As a result, hybridized bands were visualized in the length of 7.5 kilobases (kb) against the probe prepared from pSOD614 #2, and in the length of 8.0 kilobases (kb) against the probe prepared from pSOD614 #3.

### Example 5: Cloning of genomic fragments containing SOD genes whose sequences were different from each other

Four µg of the genomic DNA was digested by *EcoR*I and subjected to agarose gel electrophoresis. Then, DNAs whose length are within the range from 7 to 9 kb were recovered by a conventional elution method by using dialysis membrane. The purified DNA was ligated to 1 µg of *EcoR*I-digested and CIAP (calf intestine alkaline phosphatase) - treated λZAPII (Stratagene) at 16 °C overnight, and packaged with Gigapack III gold packaging extract (Stratagene). The packaged extract was infected to *E*. *coli* XLlBlue MRF' strain and over-laid with NZY medium poured onto LB agar medium. About 6000 plaques were screened by using *EcoR*I- digested pSOD614 #2 and pSOD614 #3 as probes. Six plaques were hybridized to the labeled pSOD614 #2 probe and two plaques were hybridized to the labeled pSOD614 #3 probe. Then, hybridized plaques were subjected to *in viv*o excision protocol according to the method specified by the manufacturer (Stratagene). It was found that one plasmid isolated from six of pSOD614 #2-hybridized plaques had the same fragments as that of pSOD614 #2 as a result of PCR analysis by using sodl and sod4 primers. This plasmid was named as pSOD703. It was also found that two plasmids isolated from two of pSOD614 #3-hybridized plaques had the same fragment as that of pSOD614 #3 as a result of PCR analysis by using sod1 and sod4 primers. One of the plasmids was named as pSOD626 and used for further study.

### Example 6: Sequence analysis of two species of MnSOD genes obtained from P. rhodozyma

A complete nucleotide sequence was determined by sequencing of pSOD703 and pSOD626 with a primer-walking procedure. Nucleotide and their deduced amino acid sequences for *SOD1* gene which harbored on pSOD703 and for *SOD2* gene which harbored on pSOD626 are listed as SEQ ID NO: 1, NO: 2, NO: 5 and NO: 7 in the sequence listing section.

Both of deduced amino acid sequences of *SODl* and *SOD2* genes were homologous to known MnSODs obtained from other species and not to Cu/ZnSODs or FeSOD as a result of BLAST analysis (Altschul, S.F. *et al*., *J. Mol. Biol.* 215, 403-410, 1990).

*SOD1* gene had 7 introns and 8 exons. And its deduced open reading frame consisted of 223 amino acids. On the other hand, *SOD*2 gene had 10 introns and 11 exons, and its deduced open reading frame consisted of 199 amino acids. Most difference between two isolated *SOD* genes was extended region of *SOD*1 gene in its amino terminal end, whose sequence might act as a transit peptide to mitochondria

In fact, Schroeder et *al*. reported two species of SODs which were detected as KCNand H₂O₂ resistant SODs in activity staining of native polyacrylamide gel electrophoresis (PAGE) in *P. rhodozyma*. They commented two species of MnSOD were indicated as aggregates or isozymes and did not reffer to their precise nature and their subcellular location. As described in the following section, it was clarified that two species of these KCN- and H₂O₂₋ resistant SODs (i.e. MnSOD) were products of *SOD1* and *SOD2* genes. As described in the section of "detailed description of the invention", most eucaryotes have different species of SODs in their intracellular location (MnSOD in the mitochondrial fraction and Cu/ZnSOD in the cytoplasmic fraction). This is the first example in which two species of MnSODs functioned in a different subcellular location.

### Example 7: Construction of disruption plasmids for SOD1 and SOD2 genes

As described in the section "detailed description of the invention", a plasmid harboring a drug resistant marker cassette was constructed by inserting G418 resistant structure gene between promoter and terminator genes of glyceraldehyde-3-phosphate dehydrogenase (GAP) and ligating into *Kpn*I- and *Hind*III- digested pUC19. This plasmid was named as pUC-G418 and used for a further study. As gene fragments which were used for homologous recombination, partial fragments *of SOD1* and *SOD2* genes were synthesized *in vitro* by PCR method using PCR primers whose sequences were shown in TABLE 2.

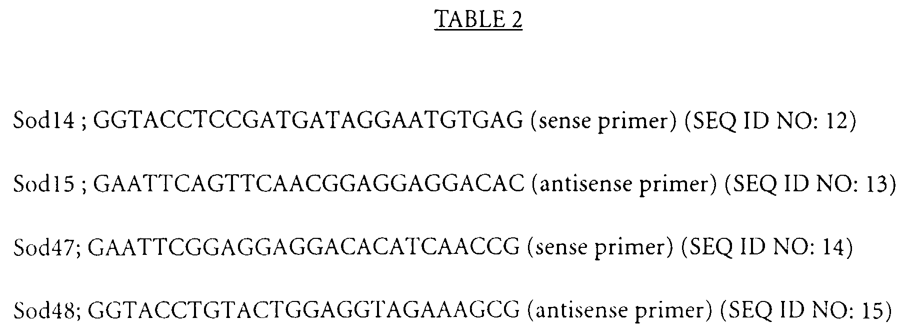

PCR condition was as follows; 25 cycles of 94 °C for 15 seconds, 50 °C for 30 seconds and 72 °C for 15 minutes. As a template, 0.1 µg of genomic DNA obtained in Example 3 was used, and ExTaq as a DNA polymerase. A partial fragment of *SOD1* which could be obtained from PCR using sodl4 and sod15 as primers and a partial *SOD*2 gene which could be obtained from PCR using sod47 and sod48 as primers were amplified respectively. Each amplified 0.65 kb fragments was recovered and cloned in pCR2.1-TOPO (Invitrogen) according to the protocol specified by the manufacturer. Six independent clones from white colonies *of E*. *coli* TOP10 transformants were selected and plasmids were prepared from those transformants. As a result of restriction analysis and sequencing, one clone which had a partial *SOD1* gene was selected for a further study and named as pSOD715. In a similar manner, one clone which had a partial *SOD2* gene was selected and named as pSOD826.

Then, pSOD715 and pSOD826 were digested by *EcoR*I and *Kpn*I, and 0.65kb fragments yielded were purified by using QIAquick protocol and ligated to *EcoRI-* and *KpnI*- digested pUC19-G418. Six independent clones from ampicillin-resistant colonies of *E*. *coli* DH5α transformants were selected and plasmids were prepared from those transformants. As a result of restriction analysis and sequencing, one clone in which a partial *SOD1* was fused to G418 resistant cassette was obtained and named as pSOD/G717. In a similar manner, one clone in which a partial *SOD2* was fused to G418 resistant cassette was obtained and named as pSOD/G828.

### Example 8: Transformation of P. rhodozyma ATCC96594 with biolistic method

Transformation protocol were followed to the method described in Methods in Molecular Biology (Johnston *et al.,* 53; 147-153, 1996). As a host strain, *P. rhodozyma* ATCC 96594 was cultured in YPD medium to stationary phase. After centrifugation of broth, cells were concentrated by 10-fold with sterilized water and 200 µl of cell suspension were spread on YPD medium containing 100 µg of geneticin, and 0.75 M of mannitol and sorbitol. Five micrograms of circular DNA of pSOD/G717 and pSOD/G828 were coated on 1.5 mg of 0.9 µm gold particle, and used as donor DNA for biolistic transformation. Hundreds of geneticin-resistant clones were yielded after one week of incubation at 20 °C. Four of those transformants were selected and chromosomes were prepared from them. One of the transformants was confirmed to have disrupted structure of chromosomal *SOD1* or *SOD2* gene by PCR and Southern blot hybridization analyses, and was used for further study.

### Example 9: Activity staining of native PAGE by using crude extracts which obtained from candidates for SOD1 and SOD2 disruptants

ATCC96594 strain and candidates which obtained from biolistic transformation of ATCC96594 were cultivated in YPD medium for two days and harvested by centrifugation for 10 min. by 3000 x g at 4 °C. After wash with Tris-HCl buffer (10mM / pH 8.0), cells were concentrated by 10-fold with the same buffer. Cells were disrupted with French press homogenizer (Ohtake Works) at 1500 kg/cm² and crude extract was prepared after a microcentrifugation at 15000 rpm (TOMY, MRX150) of homogenized fraction.

Protein concentration of crude extract thus prepared was determined with BCA protein assay reagent released by PIERCE (Rockford, U.S.A.). A volume of crude extract corresponding to 300 µg of protein were subjected to native PAGE according to the method described by Schroeder W. A. *et al*. *(J. Gen*. *Microbiol*., 139, 907-912, 1993). Method of activity staining was referred to the method by Flohé *et al*. *(Methods in Enzymology*, 105, 93-104, 1984).

Result of activity staining is depicted in FIG. 1. In the extract of parental strain, ATCC96594, two bands were visualized as transparent bands which have SOD activity in the dark background. On the contrary, ATCC96594 :: pSOD/G717 strain in which *SOD1* gene was disrupted, lacked activity band with high mobility in native PAGE and ATCC96594 :: pSOD/G828 strain, in which *SOD2* gene was disrupted lacks activity band with low mobility in native PAGE. From this result, it was found that two species of MnSOD that were present in the crude extract of *P. rhodozyma* were products *of SOD1* and *SOD2* genes, and SOD species with high mobility and with low mobility in native PAGE corresponded to *SOD1* and *SOD2* gene product, respectively.

### Example 10: Cloning of partial catalase (CAT) gene from P. rhodozyma

To clone a partial *CAT* gene from *P*. *rhodozyma,* a degenerate PCR method was exploited. Two mixed primers whose nucleotide sequences were designed and synthesized as shown in TABLE 3 based on the common sequence of known catalase genes from other species.

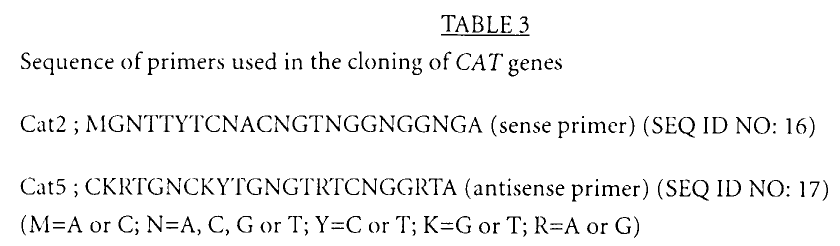

After the PCR reaction of 25 cycles of 94 °C for 15 seconds, 45 °C for 30 seconds and 72°C for 15 seconds by using ExTaq (Takara Shuzo) as a DNA polymerase and genomic DNA obtained in example 3 as a template, reaction mixture was applied to agarose gel electrophoresis. A PCR band that has a 1.0 kb length was recovered and purified by QIAquick (QIAGEN) according to the method by the manufacturer and then ligated to pCR2.1-TOPO (Invitrogen). After the transformation of competent *E. coli* TOP 10, 6 white colonies were selected and plasmids were isolated with Automatic DNA isolation system. As a result of sequencing, it was found that two clones had sequence whose deduced amino acid sequences was similar to known *CAT* genes. One of these isolated DNA clones was designated as pCAT702 and used for further study.

### Example 1 Cloning of genomic fragments containing CAT gene

In a similar manner to Example 4, Southern blot hybridization study was performed by using pCAT702 as a probe. As a result, hybridized band which had a size from 9 kb to 23 kb was visualized. Next, 4 µg of the genomic DNA was digested by *EcoR*I and subjected to agarose gel electrophoresis. Then, DNAs whose length is within the range from 9 to 23 kb was recovered by a conventional elution method by using dialysis membrane. The purified DNA was ligated to 1 µg of *EcoR*I-digested and CIAP (calf intestine alkaline phosphatase) - treated λDASHII (Stratagene) at 16 °C overnight, and packaged by Gigapack III gold packaging extract (Stratagene). The packaged extract was infected to *E*. *coli* XL1Blue MRA(P2) strain and over-laid with NZY medium poured onto LB agar medium. About 8000 plaques were screened using *EcoR*I- digested pCAT702 as a probe. Six plaques were hybridized to the labeled pCAT702 probe. λDNA was prepared from each λ clone and it was found that 4 of 6 clones contained same fragment to the insert of pCAT702 as a result of PCR using Cat2 and Cat5 primers, and sequencing analysis. A partial nucleotide and its deduced amino acid sequence for *CAT* gene are listed as SEQID NO: 3 and SEQID NO: 9 in the sequence listing section.

### Example 12 Construction of disruption plasmid for CAT gene

In a similar manner to Example 7, disruption plasmid for *CAT* gene was constructed. At first, *Sac*I linker was inserted at *Hind*III site of pUC19-G418, in which terminator region of G418-resistant cassette was located, and as a result of restriction analysis, pUCl9-G418Sa, which had *Sac*I site at the end of the G418-resistant cassette was yielded. Then, *Kpn*I- and *Sac*I- fragment derived from pUCl9-G419Sa was ligated to *Kpn*I- and *Sac*I- digested pCAT702 and yielded pCAT/G706, in which a partial genomic *CAT* gene was fused to G418-resistant cassette.

### Example 13 Transformation of P. rhodozyma ATCC 96594 by using pCAT/G706 as donor plasmid

In a same manner to Example 8, *P*. *rhodozyma* ATCC 96594 was transformed with *CAT*-disruption plasmid, pCAT/G706. Hundreds of geneticin-resistant clones were yielded after one week of incubation at 20 °C. Four of those transformants were selected and chromosomes were prepared from them. One of the transformants was confirmed to have disrupted structure of chromosomal *CAT* gene by PCR and Southern blot hybridization analyses, and was used for further study.

Then, two candidates for *CAT* disruptant were characterized with catalase test, which was often used in bacterial taxonomic study. One loopful of cells of *P. rhodozyma* was soaked in 3% H₂O₂ solution and then occurrence of dioxygen gas was observed. Although an immediate occurrence of O₂ foam were confirmed when ATCC 96594 cells were applied to this catalase test, O₂ foam was occurred after a long lag when two ATCC 96594 :: pCAT/G706 mutants were soaked in H₂O₂ solution. From this result, disruption of *CAT* gene was suggested, but a remaining weak activity indicated a presence of another player who catalyzes disappearance of H₂O₂ such as peroxidase in *P*. *rhodozyma*.

### Example 14 Evaluation of SOD1, SOD2 and CAT disruptants derived from P. rhodozyma for their astaxanthin production

Effect of gene disruption of *SOD*1, *SOD*2 and *CAT* gene on astaxanthin production was evaluated by cultivation in YPD medium with shaking flasks. Cells which grew on YPD agar were suspended in YPD medium and a portion of cell suspension was inoculated to 50 ml of YPD medium in 500 ml baffled flask. Cells were grown with 200 r. p. m. at 20 °C for 84 hours. At an appropriate interval, 3 ml of broth was withdrawn and was analyzed for cell yield, consumption of glucose and astaxanthin content.

Cell yield was measured as optical density at 660 nm and as dry cell weight by weighing cells after filtration through 0.45 µm cellulose acetate plus nitrocellulose membrane (HAWP04700, Millipore, Bedford, U.S.A.) and heating at 80 °C overnight. Astaxanthin content of *P*. *rhodozyma* was measured with HPLC method after extraction of carotenoids from cells of *P. rhodozyma* by disruption with glass beads. After extraction, 5ml of acetone/BHT/water containing appropriate concentration ofbixin as an internal standard was added. Supernatant was analyzed for astaxanthin content with following HPLC system.
HPLC column; YMC-Pak ODS-A (6 mm, 150 mm)
Temperature; room temperature
Eluent; acetonitrile / methanol / isopropanol (85/10/5)
Injection volume; 10 µl
Flow Rate; 2.0 ml/minute
Detection; UV at 471 nm
Results obtained from 84 hour-culture are summarized in TABLE 4.

**TABLE 4**

| The effect of *SOD* and *CAT* mutation on productivity of total carotenoids and astaxanthin by *P. rhodozyma* | | |
|---|---|---|
| Strain | Total carotenoids (mg/g-dry-cell) | astaxanthin (mg/g-dry cell) |
| ATCC 96594 | 0.169 | 0.111 |
| *ΔSOD1* | 0.259 | 0.146 |
| Δ*SOD2* | 0.202 | 0.129 |
| *ΔCAT* | 0.229 | 0.144 |

*SOD1* and *SOD2* disruptants showed elevated level of productivity for total carotenoids as well as astaxanthin compared to their host strain, ATCC 96594. Especially, *SOD1* disruptant showed significant increase of carotenoids and astaxanthin production by 53.3 %and 31.5 %, respectively.

SOD1 seemed to be a mitochondrial enzyme judged from deduced transit peptide sequence at its amino terminal end and might act to scavenge superoxide radical, a kind of active oxygen species occurred in the respiratory chain at mitochondria. These data suggested that astaxanthin production was stimulated by a generation of intracellular active oxygen to compensate the lack of native player of active oxygen species-quenching factor, SOD1.

SEQUENCE LISTING

<110> F. HOFFMANN-LA ROCHE AG

<120> Improvement of biological production of carotenoids and
biological materials therefor

<130> SOD

<140>
<141>

<150> EP99123821.3
<151> 1999-12-01

<160> 17

<170> PatentIn Ver. 2.0

<210> 1
<211> 3632
<212> DNA
<213> Phaffia rhodozyma

<220>
<221> 5'UTR
<222> (922)..(923)
<223> EXPERIMENTAL

<220>
<221> exon
<222> (986)..(1096)

<220>
<221> intron
<222> (1097)..(1306)

<220>
<221> exon
<222> (1307)..(1456)

<220>
<221> intron
<222> (1457)..(1555)

<220>
<221> exon
<222> (1556)..(1589)

<220>
<221> intron
<222> (1590)..(1694)

<220>
<221> exon
<222> (1695)..(1799)

<220>
<221> intron
<222> (1800)..(1920)

<220>
<221> exon
<222> (1921)..(1982)

<220>
<221> intron
<222> (1983)..(2076)

<220>
<221> exon
<222> (2077)..(2140)

<220>
<221> intron
<222> (2141)..(2246)

<220>
<221> exon
<222> (2247)..(2272)

<220>
<221> intron
<222> (2273)..(2390)

<220>
<221> exon
<222> (2391)..(2507)

<220>
<221> polyA_site
<222> (2663)..(2664)
<223> EXPERIMENTAL

<400> 1
tcctgttgat aatctttcta acgccttgta ctttgaccaa ggcgtttgtc cgaaattttg 60

caaacttagt gttggtcgca tggacggtct tcggatccag aactgacggc tcgccaataa 120

agtatgacga tggtagaggt gaaggaggga accacaggtt gaccagtctc aaagagtgct 180

gatgtgcgcg aggatttgtc attaaatggt gttgtatatg ctagagccaa gagaagacat 240

ttggttttgg ttttggtttt gcatttgatg agatgtgtca cgattgaaga cgggaggagg 300

ctcactaacc caagaagcca ggatcaggag gaatgcctcc cccttttcat caagatcttt 360

ctcacatcga acatttgaca ttctctttag tatccttcta tccttttctt ccaacttctc 420

ccattgtatc gactttgctc gacttgctct tcttatctct gagcagagat gggcattcca 480

atatcgaagg agcgacacaa gaccttggag tttgggtaac agatgaagag gggccgaggt 540

ggatggggct gtaggaagta gctgatcgat gagttcctgg atgatgatag gcgaaggaac 600

agacatagga tctctgtctc gtcctggaat tactgagtct tgtatccagc gtgttcttgt 660

ctcgaagaag ccttcaagat cgatgtaaga taagacaggc aatgaggacg gacgaacgaa 720

cgaacgaaaa gaacagaaga gctggtaagt cagtcagtca gtcagtcagt caatcaaaca 780

ctggtgtcta gggttatagc tcgacgcgac gcgacgcgtt tgagacgcga tatgcttacg 840

taatacctgg cgtcatcccc ccagccgagg caagagccga gccgctcgtg aacgacaaaa 900

ttcaaaaggc tttctccatc ttaagctcat tctcatctaa ccgactcatc tcgttcccat 960

cattcccatc attctaccgc catcc atg tct gtt cga gca tcc ctc tct tcc 1012

gtg tct aga cag act ttc gtc gct cct gct gct ttc cag atc agg gca 1060

aag cat acc ctg cct gag ctt cct tac gct tac gat gtaagacttt 1106

tccgtgttct cctattcgtc gctttcttgg tttttttcgt cttcgccctc tagctcttct 1166

tcgtcctttc tgtcctgctc tttgttgttg atattcagct cgatagacta acccatctca 1226

tctcctggac attcttttac tggaaacgta tcttgtcctt ggtttttctt ggctttggtt 1286

gaaaattcct ctccactcag gcc ctg gag ccc tcc atc tcc aag gag atc atg 1339

acc ctt cac cac acc aag cac cat cag act tat gtt aac ggc ctc aac 1387

gct gcc gag gag agc tac tcg gcc gct gtg ggc aag gag gat gtg ctt 1435

acc cag gtt aag ctt cag tct gtacgtctga ccgtttttt atcgaccgga 1486

acgcctggtg aggagggaga tgaagtttga tgagcgctca tcgtctagca cgttgacccg 1546

atcatacag gct ctc aag ttc aac gga gga gga cac atc aat c 1589

gtcagtgata ttcttcaaac tcttgctgag caagtcaggt caagctgact gtttcgcttt 1649

gtttctgcgg atctatctca tccttgattt ggcatgatga aacag ac tct ctg ttc 1705

tgg aag aac ttg gct ccc tat gga tcc gag gag gct acc ctc tct gaa 1753

gga cct ctc aag aag gct atc gag gaa tct ttt ggt tct ttc gag g 1799

gtccgtccat ctatcttcct attcagttgt gtttggttcc ggtatactca tctgttttgt 1859

ttccccacaa aataaaaata aaaatcttgt cctctccggg ggttcgactg cacgttcata 1919

g cc ttc aag aag aag ttc aac gct gac acc gct gct gtc caa gga tcc 1967

gga tgg ggc tgg ctt gtatgtatca tatcctttcc atctcaaact cttctcagag 2022

tctttttcct tgagacttca aactgactat acatgtttct acaacaaaca acag ggc 2079

ttg aac ccg ctt act aag aag ctg gaa gtc acc acg acc gcc aac cag 2127

gac cct ctg ctt a gtaagttgtt tctacatgat tttctatctc aacgcgatct 2180

gcatgattcg tcactgattc actggattct cttgtttcgt ttttctcggg atgatttcat 2240

aaacag ct cac att cct atc atc gga gtt gac gtgcgtatct ttcttgaata 2292

gtcgtagcgt ctgatctcgt tttattgact gacgtgttgc ttctgtccaa atcattaaaa 2352

aaaatgaaaa caaataatcg attgaccgac gaaaacag atc tgg gag cac gct ttc 2408

tac ctt cag tac aag aac gtc aag cct gac tat ctc gct gct gtt tgg 2456

tcc gtt atc aac tac aag gag gca gag gcc cga ttg cag gct gct ctc 2504

taa gcgggacgaa aagtaacgac atatgaaggg aggatcaaat atcgtttctt 2557

cataaacaac tttcgaggca gatgggagag tacgtacaag agaggtttgt atggagaatt 2617

gagtttgttg acggttagca ggttatgata tatgtagcta tagtctagtc taaatctgaa 2677

agaagagaac aagatggttt gtccgaagag attgagagat caagcccggt catctgatgt 2737

cgaacaaaca tgccctggtc tgccaacagt ttctagcaca ttatgaccat gttcatgtgt 2797

aaattgggaa atgagccaga aaggtttatt atctaattca ttgattcatg cgactatgga 2857

tacatatggg atttccagaa caaacagatg caacaaagca cggcattttc caaagatcga 2917

gtcctcccac aagtatgcgg caaggtttgt tgttaagaga tataaaagca gacgacaaaa 2977

caaatcgttt atcgaccctg tgcaccaaca ccgtgaccgt ttgacgagtt ggtagagttg 3037

tagttgttgc tgttcaaagg agctccagac tggacgcttc caagcttcaa caacttctcg 3097

gcagcgtcgc tgttcgggaa aagaaaaggc aaaaaggaac agagcgataa gcatatgtga 3157

ttctctactt cttataggct cttagctcaa gtcaactcac atgtctttgg cggtaccgaa 3217

gacgttctca agctgctgct tggaagcttt tccgagcttg ccagtaggtc cctggttgga 3277

gaagaagatg tcgaaggcta agggcgatga aaagcatgaa gatattagct atcggcgcga 3337

taaaagtgtg acgagatgaa aatggagaaa agatgattcg caccatcgac gacctcgacc 3397

aaaggaatgg aggtgtcacc ggccttccac ttcttgtact cctcaacgtt gacgaagatg 3457

acgaagcagt cggtggcctt agcctcgggc tcgtagatgc tgatgaaaca caataggtag 3517

taggagagga gaaagagaag atgatgagat gtcaggatgc ttgcttcact gtagatggag 3577

gaagaagata tgcgaagcaa gacatacact ttggaaagag cttgaaccat tgtag 3632

<210> 2
<211> 3375
<212> DNA
<213> Phaffia rhodozyma

<220>
<221> 5'UTR
<222> (974)..(975)
<223> EXPERIMENTAL

<220>
<221> exon
<222> (1040)..(1063)

<220>
<221> intron
<222> (1064)..(1237)

<220>
<221> exon
<222> (1238)..(1280)

<220>
<221> intron
<222> (1281)..(1358)

<220>
<221> exon
<222> (1359)..(1402)

<220>
<221> intron
<222> (1403)..(1483)

<220>
<221> exon
<222> (1484)..(1589)

<220>
<221> intron
<222> (1590)..(1674)

<220>
<221> exon
<222> (1675)..(1826)

<220>
<221> intron
<222> (1827)..(1907)

<220>
<221> exon
<222> (1908)..(1922)

<220>
<221> intron
<222> (1923)..(1992)

<220>
<221> exon
<222> (1993)..(2056)

<220>
<221> intron
<222> (2057)..(2131)

<220>
<221> exon
<222> (2132)..(2157)

<220>
<221> intron
<222> (2158)..(2238)

<220>
<221> exon
<222> (2239)..(2293)

<220>
<221> intron
<222> (2294)..(2376)

<220>
<221> exon
<222> (2377)..(2426)

<220>
<221> intron
<222> (2427)..(2524)

<220>
<221> exon
<222> (2525)..(2542)

<220>
<221> polyA_site
<222> (2667)..(2668)
<223> EXPERIMENTAL

<400> 2
cttatccttc tgccgctggt ctctgtctgt cgagtgtgtg ggatggtttt ggatatgttc 60

ctatacgaaa ggtagcgcag agcaaagctg acagtattaa gcaagacaag agcttctttc 120

tgttgacaga tgaaaggacg aactatgaag ctgtccatgc tccccaaacc gattgacaca 180

ccgccgtcag gcaacgcaga atttctcact gcttcgacgt cacaccaaca tcgatcctcc 240

atacctaaaa gcagatcgag acacattgtt ggtcgccatg ttggatggat gtacatcaaa 300
cccacagcat atatcactca catgtgagaa ctccgtagcc tctaccttct tgtctctcaa 360
tctgaatgtc tcgttgagag gtggaatgaa tgtttacagt ttgagaagac gaaagaaaga 420
aagagaagag aagagaggaa tacgtacgac gaagttatca tcgtatggga acttttctaa 480
aaaactgcct atagtagaga cgatctctgg aggaaagctc tgtagtatga tagtgaagag 540
cgagcaagtc tgggcaagtg catccttcgt ctacaagaaa gagaccagga aatgaaggag 600
agacgagtaa gcaggtacct accgatattg gatcgttctc tctacccagc gatgccttca 660
ccacgcgttc tatctcttct tgggatggca gatacatact taacgagagc aatctgatgt 720
ataccgaact tcgaacggaa tgatcccaga atcctcttga acccttgaac ccttgaaccc 780
tggaaccaag taccaaccga gcaacacgcc gatacggtcc acaccacaga accacacgcc 840
ctcgtcatta aaggtgggac gcgccgatgc tggttacgtt cggcccaatc cggaagttac 900
cggcttggac gtgcctgtaa ccatgccctg acggtatttc gccttcagct aactccatct 960
catctttttc ctttactacc acaacccacc cttgaacctt cttccccggc ttttttacta 1020
tatccatcta tcaatcatc atg gct cct tac act ctt ccc gac gtaagcttaa 1073
agtttgagct gtgtgtgctt atctcaatct tggagttgaa ctcaccgttt tttgtttttg 1133
cttcctggtt tttttatcgg catccctcct ttttttcccc tcgtggtcgc atatgatttg 1193
ctcatcaatc ggcgtttccc atgcatcttt gtcatccgtt tcag ctt cct tac gct 1249
tac gat gcc ttg gag cct tac atc tct aag g gtgagattct tagtcagact 1300
gttgttccgg ttcgacacga tagctaatcg tctctcgttc ctcaatatga acatgcag 1358
aa atc atg atc ctt cac cac tcc aag cac cat cag act tac gtc 1402
gtacgtaatc taaaggtcat ctccgtctac atggccggat caacttgctc atagatcttc 1462
cttctgttcg gcgctacgta g acc aac ctc aac gcc gct atc cag gct ttc 1513
tcc cag acc aat gac atc aag gcc cag atc gct ctt cag agc gct ctc 1561
aag ttc aac gga gga gga cac atc aac c gtacgatcat tctccctctt 1609
ctggcttatc atatgtgttg cttgtcacta acacgcatgc aaccccggga tatctcaccc 1669
tgtag ac tcc ctc ttc tgg aag aac atg gct cct gcc gac tct gct gat 1718
gcc aag ctc acc gag gga tcg ctc aag act gcc atc gac aag gac ttt 1766
gga tcc ttc gag gag ttc aag aag aag ttc aac act gct act ctc ggt 1814
gtc cag gga tct gtcagtatct cgtttgcttc gacatactct cagctttcct 1866
tccgtaaact gacgaatagt tttcggaca tgtacttgta g gga tgg gga tgg ctc 1922
gtgcgtttga cctttttcca ctttgaacat tagcgatagt gatacctaac aactgtgaat 1982
tggaatatag gga tac aac acc gct acc aag cac ctc gag atc gcc acc 2031
acc gcc aac cag gat ccc ctt atc a gtatgtgact tctctcgtgt 2076

ggtcaccata agccagttgc tgacacattt cgttcgctgt ctctcgactt cgtag ct   2133

ttg act ccc atc att ggt ctt gac gttagtaatt ctatctagtg attggagtcg  2187

agttctgaac ttgccttgat ctcaaacgaa tgaatcaatt tcttttggta g atc tgg  2244

gag cac gct ttc tac ctc cag tac aag aat gtc aag cct gat tac ctt g 2293

gtacgtaatt ctctattcgt ttgccccggt ttgatctttg actcactctt caaaatgttt 2353

tcgtttgta actttgaaaa acag cc gct ttc tgg aac gtc tgc aac ttt gct  2405

gag gct cag cga agg ttt gat gtgagtacag gcgctacccc tacggaggaa      2456

gcgaaggtga gctgaccact ttttatcttt ctgatttgga atgaacgatc cgatgatcaa 2516

acaaacag gct gct gtc aag gct taa tggtcccatt tatctctttg attcgacggc 2572

gatgacggct ttctcgcatc cgaagaaggc aaggctatga ttactgttat tctgccatgt 2632

tgcttgcttt gctatgctct atgttctttt cttttgcctc tcttcaaagc caaggcgtta 2692

aggaaggccc ttcagtctgt tttacatatg cacatataca tgagaacata tcacggactc 2752

ggcggctggt ggtcctcttg agcgtcggct tcaagattag tgtccacacg tgaagcgttc 2812

ggtgccatcc aacctggtag gaatccccat cgggcgggaa tccaattatc aattggcggt 2872

cggccagatt cgagctcggg tatctcagaa gcgtcaagcg ggcgcatttc caggccttta 2932

agaggagcaa atttaatccg cctgggtgtt cagcgagaca cgaacagttt gaaacagagt 2992

ctgcttgtga gttactcggc gagatcactg aggactaaac tttctcagct cgtggacgaa 3052

aagaacgaac caaacggtct tccctgtatc tcgaccatct ccttctccat ctcttacaac 3112

acctcggatg aactccaagg cttgctttcc aaagttcaaa caaactccgg gttgccatcc 3172

acctggtttg tctctaacga gccgagggat atccatcgtt cggaacgttt gaacagactg 3232

gatggtaggt ggccggtcgc ttcggaagcc aatcataatg gtgggaatcg agagaaggaa 3292

tgattgggcc cagtgtttaa gacttgtgtt tgttggcaga gtacggacgg aaagtaggac 3352

agacttaatc aaggcgagcc aag                                         3375

<210> 3
<211> 951
<212> DNA
<213> Phaffia rhodozyma

<220>
<221> exon
<222> (1)..(34)

<220>
<221> intron
<222> (35)..(115)

<220>
<221> exon
<222> (117)..(159)

<220>
<221> intron

<222> (160)..(242)

<220>
<221> exon
<222> (243)..(363)

<220>
<221> intron
<222> (364)..(436)

<220>
<221> exon
<222> (437)..(951)

<400> 3
tcc gga agc tca gat acc gct cga gat cct cga g gtttgtgtgc 44

tttcgctttg ttcgcatgga tgaagctgtt aacttaaaaa aatcctcgtg tttctctttg 104

tttcaacata g gt ttc tct ctt aag gtc aag acc tct gag gga aac tgg 153

gac ttt gtacgtattc ttatcgactg agtcatcaag ctcgttatcg ctctcttacc 209

ctcatccttt tgtgtctctg tctacacctc tag gtc gga aac aac act ccc atc 263

ttt ttc ttg aga gac cca gcc aag ttt ccg atc ttc att cac acc cag 311

aag agg aac ccg cag aca aac tct aaa gac aag gac gct ttc tgg gac 359

tac c gttcgtata accttgtcac tccctgcgtg ccgctctgat tcatgttgac 412

cttgtctttg atataatttt atag ta tcc caa aac ccc gag tcc gtg cat cag 465

gtg ctg cac ctg ttc agt gat cga gga acc cct gct tct tac cga cac 513

atg cat ggt tac tct gga cac acc ttc aag atg gtc aac agg aac ggt 561

gac tgg aat tat gtc cag att cac atg cgc acc gat cag ggt gtc aag 609

act cac acc aat gaa gag gct tcg aaa ctc gac gcc tcc aat ccc gat 657

tca aac gga gac gac ttg ttc gac gca atc aag aat gga gac ttc cct 705

agc tgg acg gtt cag gta cag gta atg tct cct gag cag gcc cag aag 753

ttc aga tac aac att ctg gat ctc acc aag gtc tgg tcc cac aag gag 801

ttc cca ctt agg acg att gga aag ttc act ttg aac cga aac gtg gat 849

aac tat ttc gca gag gtt gaa cag ctc gcc ttt gct cct tcc cat ctg 897

cct cct gga atc gag ccc tcg aac gat ccc gtc ctt cag gct cga cta 945

ttc tcc 951

<210> 4
<211> 669
<212> DNA
<213> Phaffia rhodozyma

<220>
<221> CDS
<222> (1)..(669)

<400> 4

atg tct gtt cga gca tcc ctc tct tcc gtg tct aga cag act ttc gtc     48
Met Ser Val Arg Ala Ser Leu Ser Ser Val Ser Arg Gln Thr Phe Val
  1               5                  10                  15

gct cct gct gct ttc cag atc agg gca aag cat acc ctg cct gag ctt     96
Ala Pro Ala Ala Phe Gln Ile Arg Ala Lys His Thr Leu Pro Glu Leu
             20                  25                  30

cct tac gct tac gat gcc ctg gag ccc tcc atc tcc aag gag atc atg    144
Pro Tyr Ala Tyr Asp Ala Leu Glu Pro Ser Ile Ser Lys Glu Ile Met
         35                  40                  45

acc ctt cac cac acc aag cac cat cag act tat gtt aac ggc ctc aac    192
Thr Leu His His Thr Lys His His Gln Thr Tyr Val Asn Gly Leu Asn
     50                  55                  60

gct gcc gag gag agc tac tcg gcc gct gtg ggc aag gag gat gtg ctt    240
Ala Ala Glu Glu Ser Tyr Ser Ala Ala Val Gly Lys Glu Asp Val Leu
 65                  70                  75                  80

acc cag gtt aag ctt cag tct gct ctc aag ttc aac gga gga gga cac    288
Thr Gln Val Lys Leu Gln Ser Ala Leu Lys Phe Asn Gly Gly Gly His
                 85                  90                  95

atc aat cac tct ctg ttc tgg aag aac ttg gct ccc tat gga tcc gag    336
Ile Asn His Ser Leu Phe Trp Lys Asn Leu Ala Pro Tyr Gly Ser Glu
            100                 105                 110

gag gct acc ctc tct gaa gga cct ctc aag aag gct atc gag gaa tct    384
Glu Ala Thr Leu Ser Glu Gly Pro Leu Lys Lys Ala Ile Glu Glu Ser
        115                 120                 125

ttt ggt tct ttc gag gcc ttc aag aag aag ttc aac gct gac acc gct    432
Phe Gly Ser Phe Glu Ala Phe Lys Lys Lys Phe Asn Ala Asp Thr Ala
    130                 135                 140

gct gtc caa gga tcc gga tgg ggc tgg ctt ggc ttg aac ccg ctt act    480
Ala Val Gln Gly Ser Gly Trp Gly Trp Leu Gly Leu Asn Pro Leu Thr
145                 150                 155                 160

aag aag ctg gaa gtc acc acg acc gcc aac cag gac cct ctg ctt act    528
Lys Lys Leu Glu Val Thr Thr Thr Ala Asn Gln Asp Pro Leu Leu Thr
                165                 170                 175

cac att cct atc atc gga gtt gac atc tgg gag cac gct ttc tac ctt    576
His Ile Pro Ile Ile Gly Val Asp Ile Trp Glu His Ala Phe Tyr Leu
            180                 185                 190

cag tac aag aac gtc aag cct gac tat ctc gct gct gtt tgg tcc gtt    624
Gln Tyr Lys Asn Val Lys Pro Asp Tyr Leu Ala Ala Val Trp Ser Val
        195                 200                 205

atc aac tac aag gag gca gag gcc cga ttg cag gct gct ctc taa        669
Ile Asn Tyr Lys Glu Ala Glu Ala Arg Leu Gln Ala Ala Leu
    210                 215                 220


<210> 5
<211> 222
<212> PRT
<213> Phaffia rhodozyma

<400> 5
Met Ser Val Arg Ala Ser Leu Ser Ser Val Ser Arg Gln Thr Phe Val
  1               5                  10                  15

Ala Pro Ala Ala Phe Gln Ile Arg Ala Lys His Thr Leu Pro Glu Leu
            20                  25                  30

Pro Tyr Ala Tyr Asp Ala Leu Glu Pro Ser Ile Ser Lys Glu Ile Met
        35                  40                  45

Thr Leu His His Thr Lys His His Gln Thr Tyr Val Asn Gly Leu Asn
    50                  55                  60

Ala Ala Glu Glu Ser Tyr Ser Ala Ala Val Gly Lys Glu Asp Val Leu
65                  70                  75                  80

Thr Gln Val Lys Leu Gln Ser Ala Leu Lys Phe Asn Gly Gly Gly His
                85                  90                  95

Ile Asn His Ser Leu Phe Trp Lys Asn Leu Ala Pro Tyr Gly Ser Glu
            100                 105                 110

Glu Ala Thr Leu Ser Glu Gly Pro Leu Lys Lys Ala Ile Glu Glu Ser
        115                 120                 125

Phe Gly Ser Phe Glu Ala Phe Lys Lys Lys Phe Asn Ala Asp Thr Ala
    130                 135                 140

Ala Val Gln Gly Ser Gly Trp Gly Trp Leu Gly Leu Asn Pro Leu Thr
145                 150                 155                 160

Lys Lys Leu Glu Val Thr Thr Thr Ala Asn Gln Asp Pro Leu Leu Thr
                165                 170                 175

His Ile Pro Ile Ile Gly Val Asp Ile Trp Glu His Ala Phe Tyr Leu
            180                 185                 190

Gln Tyr Lys Asn Val Lys Pro Asp Tyr Leu Ala Ala Val Trp Ser Val
        195                 200                 205

Ile Asn Tyr Lys Glu Ala Glu Ala Arg Leu Gln Ala Ala Leu
    210                 215                 220

<210> 6
<211> 597
<212> DNA
<213> Phaffia rhodozyma

<220>
<221> CDS
<222> (1)..(597)

<400> 6
atg gct cct tac act ctt ccc gac ctt cct tac gct tac gat gcc ttg      48
Met Ala Pro Tyr Thr Leu Pro Asp Leu Pro Tyr Ala Tyr Asp Ala Leu
1               5                   10                  15

gag cct tac atc tct aag gaa atc atg atc ctt cac cac tcc aag cac      96
Glu Pro Tyr Ile Ser Lys Glu Ile Met Ile Leu His His Ser Lys His
            20                  25                  30

cat cag act tac gtc acc aac ctc aac gcc gct atc cag gct ttc tcc      144
His Gln Thr Tyr Val Thr Asn Leu Asn Ala Ala Ile Gln Ala Phe Ser
        35                  40                  45

cag acc aat gac atc aag gcc cag atc gct ctt cag agc gct ctc aag      192
Gln Thr Asn Asp Ile Lys Ala Gln Ile Ala Leu Gln Ser Ala Leu Lys
    50                  55                  60

ttc aac gga gga gga cac atc aac cac tcc ctc ttc tgg aag aac atg     240
Phe Asn Gly Gly Gly His Ile Asn His Ser Leu Phe Trp Lys Asn Met
65                  70                  75                  80

gct cct gcc gac tct gct gat gcc aag ctc acc gag gga tcg ctc aag     288
Ala Pro Ala Asp Ser Ala Asp Ala Lys Leu Thr Glu Gly Ser Leu Lys
                85                  90                  95

act gcc atc gac aag gac ttt gga tcc ttc gag gag ttc aag aag aag     336
Thr Ala Ile Asp Lys Asp Phe Gly Ser Phe Glu Glu Phe Lys Lys Lys
            100                 105                 110

ttc aac act gct act ctc ggt gtc cag gga tct gga tgg gga tgg ctc     384
Phe Asn Thr Ala Thr Leu Gly Val Gln Gly Ser Gly Trp Gly Trp Leu
        115                 120                 125

gga tac aac acc gct acc aag cac ctc gag atc gcc acc acc gcc aac     432
Gly Tyr Asn Thr Ala Thr Lys His Leu Glu Ile Ala Thr Thr Ala Asn
    130                 135                 140

cag gat ccc ctt atc act ttg act ccc atc att ggt ctt gac atc tgg     480
Gln Asp Pro Leu Ile Thr Leu Thr Pro Ile Ile Gly Leu Asp Ile Trp
145                 150                 155                 160

gag cac gct ttc tac ctc cag tac aag aat gtc aag cct gat tac ctt     528
Glu His Ala Phe Tyr Leu Gln Tyr Lys Asn Val Lys Pro Asp Tyr Leu
                165                 170                 175

gcc gct ttc tgg aac gtc tgc aac ttt gct gag gct cag cga agg ttt     576
Ala Ala Phe Trp Asn Val Cys Asn Phe Ala Glu Ala Gln Arg Arg Phe
            180                 185                 190

gat gct gct gtc aag gct taa                                         597
Asp Ala Ala Val Lys Ala
        195


<210> 7
<211> 198
<212> PRT
<213> Phaffia rhodozyma

<400> 7
Met Ala Pro Tyr Thr Leu Pro Asp Leu Pro Tyr Ala Tyr Asp Ala Leu
1               5                   10                  15

Glu Pro Tyr Ile Ser Lys Glu Ile Met Ile Leu His His Ser Lys His
            20                  25                  30

His Gln Thr Tyr Val Thr Asn Leu Asn Ala Ala Ile Gln Ala Phe Ser
        35                  40                  45

Gln Thr Asn Asp Ile Lys Ala Gln Ile Ala Leu Gln Ser Ala Leu Lys
    50                  55                  60

Phe Asn Gly Gly Gly His Ile Asn His Ser Leu Phe Trp Lys Asn Met
65                  70                  75                  80

Ala Pro Ala Asp Ser Ala Asp Ala Lys Leu Thr Glu Gly Ser Leu Lys
                85                  90                  95

Thr Ala Ile Asp Lys Asp Phe Gly Ser Phe Glu Glu Phe Lys Lys Lys
            100                 105                 110

Phe Asn Thr Ala Thr Leu Gly Val Gln Gly Ser Gly Trp Gly Trp Leu

115                 120                 125

Gly Tyr Asn Thr Ala Thr Lys His Leu Glu Ile Ala Thr Thr Ala Asn
    130                 135                 140

Gln Asp Pro Leu Ile Thr Leu Thr Pro Ile Ile Gly Leu Asp Ile Trp
145                 150                 155                 160

Glu His Ala Phe Tyr Leu Gln Tyr Lys Asn Val Lys Pro Asp Tyr Leu
                165                 170                 175

Ala Ala Phe Trp Asn Val Cys Asn Phe Ala Glu Ala Gln Arg Arg Phe
            180                 185                 190

Asp Ala Ala Val Lys Ala
        195

<210> 8
<211> 714
<212> DNA
<213> Phaffia rhodozyma

<220>
<221> CDS
<222> (1)..(714)

<400> 8
tcc gga agc tca gat acc gct cga gat cct cga ggt ttc tct ctt aag      48
Ser Gly Ser Ser Asp Thr Ala Arg Asp Pro Arg Gly Phe Ser Leu Lys
  1               5                  10                  15

gtc aag acc tct gag gga aac tgg gac ttt gtc gga aac aac act ccc      96
Val Lys Thr Ser Glu Gly Asn Trp Asp Phe Val Gly Asn Asn Thr Pro
             20                  25                  30

atc ttt ttc ttg aga gac cca gcc aag ttt ccg atc ttc att cac acc     144
Ile Phe Phe Leu Arg Asp Pro Ala Lys Phe Pro Ile Phe Ile His Thr
         35                  40                  45

cag aag agg aac ccg cag aca aac tct aaa gac aag gac gct ttc tgg     192
Gln Lys Arg Asn Pro Gln Thr Asn Ser Lys Asp Lys Asp Ala Phe Trp
     50                  55                  60

gac tac cta tcc caa aac ccc gag tcc gtg cat cag gtg ctg cac ctg     240
Asp Tyr Leu Ser Gln Asn Pro Glu Ser Val His Gln Val Leu His Leu
 65                  70                  75                  80

ttc agt gat cga gga acc cct gct tct tac cga cac atg cat ggt tac     288
Phe Ser Asp Arg Gly Thr Pro Ala Ser Tyr Arg His Met His Gly Tyr
                 85                  90                  95

tct gga cac acc ttc aag atg gtc aac agg aac ggt gac tgg aat tat     336
Ser Gly His Thr Phe Lys Met Val Asn Arg Asn Gly Asp Trp Asn Tyr
            100                 105                 110

gtc cag att cac atg cgc acc gat cag ggt gtc aag act cac acc aat     384
Val Gln Ile His Met Arg Thr Asp Gln Gly Val Lys Thr His Thr Asn
        115                 120                 125

gaa gag gct tcg aaa ctc gac gcc tcc aat ccc gat tca aac gga gac     432
Glu Glu Ala Ser Lys Leu Asp Ala Ser Asn Pro Asp Ser Asn Gly Asp
    130                 135                 140

gac ttg ttc gac gca atc aag aat gga gac ttc cct agc tgg acg gtt     480

Asp Leu Phe Asp Ala Ile Lys Asn Gly Asp Phe Pro Ser Trp Thr Val
145                 150                 155                 160

cag gta cag gta atg tct cct gag cag gcc cag aag ttc aga tac aac     528
Gln Val Gln Val Met Ser Pro Glu Gln Ala Gln Lys Phe Arg Tyr Asn
                165                 170                 175

att ctg gat ctc acc aag gtc tgg tcc cac aag gag ttc cca ctt agg     576
Ile Leu Asp Leu Thr Lys Val Trp Ser His Lys Glu Phe Pro Leu Arg
            180                 185                 190

acg att gga aag ttc act ttg aac cga aac gtg gat aac tat ttc gca     624
Thr Ile Gly Lys Phe Thr Leu Asn Arg Asn Val Asp Asn Tyr Phe Ala
        195                 200                 205

gag gtt gaa cag ctc gcc ttt gct cct tcc cat ctg cct cct gga atc     672
Glu Val Glu Gln Leu Ala Phe Ala Pro Ser His Leu Pro Pro Gly Ile
    210                 215                 220

gag ccc tcg aac gat ccc gtc ctt cag gct cga cta ttc tcc             714
Glu Pro Ser Asn Asp Pro Val Leu Gln Ala Arg Leu Phe Ser
225                 230                 235


<210> 9
<211> 238
<212> PRT
<213> Phaffia rhodozyma

<400> 9
Ser Gly Ser Ser Asp Thr Ala Arg Asp Pro Arg Gly Phe Ser Leu Lys
  1               5                  10                  15

Val Lys Thr Ser Glu Gly Asn Trp Asp Phe Val Gly Asn Asn Thr Pro
             20                  25                  30

Ile Phe Phe Leu Arg Asp Pro Ala Lys Phe Pro Ile Phe Ile His Thr
         35                  40                  45

Gln Lys Arg Asn Pro Gln Thr Asn Ser Lys Asp Lys Asp Ala Phe Trp
     50                  55                  60

Asp Tyr Leu Ser Gln Asn Pro Glu Ser Val His Gln Val Leu His Leu
 65                  70                  75                  80

Phe Ser Asp Arg Gly Thr Pro Ala Ser Tyr Arg His Met His Gly Tyr
                 85                  90                  95

Ser Gly His Thr Phe Lys Met Val Asn Arg Asn Gly Asp Trp Asn Tyr
            100                 105                 110

Val Gln Ile His Met Arg Thr Asp Gln Gly Val Lys Thr His Thr Asn
        115                 120                 125

Glu Glu Ala Ser Lys Leu Asp Ala Ser Asn Pro Asp Ser Asn Gly Asp
    130                 135                 140

Asp Leu Phe Asp Ala Ile Lys Asn Gly Asp Phe Pro Ser Trp Thr Val
145                 150                 155                 160

Gln Val Gln Val Met Ser Pro Glu Gln Ala Gln Lys Phe Arg Tyr Asn
                165                 170                 175

Ile Leu Asp Leu Thr Lys Val Trp Ser His Lys Glu Phe Pro Leu Arg
            180                 185                 190

Thr Ile Gly Lys Phe Thr Leu Asn Arg Asn Val Asp Asn Tyr Phe Ala
        195                 200                 205

Glu Val Glu Gln Leu Ala Phe Ala Pro Ser His Leu Pro Pro Gly Ile
    210                 215                 220

Glu Pro Ser Asn Asp Pro Val Leu Gln Ala Arg Leu Phe Ser
225                 230                 235


<210> 10
<211> 23
<212> DNA
<213> Artificial Sequence

<220>
<223> Description of Artificial Sequence:Sod1(sense
      primer for cloning of SOD genes)

<400> 10
aarcaycayc aracntaygt naa                                          23


<210> 11
<211> 23
<212> DNA
<213> Artificial Sequence

<220>
<223> Description of Artificial Sequence:Sod4 (antisense
      primer for cloning of SOD genes)

<400> 11
gcccanccng anccytgnac ncc                                          23


<210> 12
<211> 26
<212> DNA
<213> Artificial Sequence

<220>
<223> Description of Artificial Sequence:Sod14 (sense
      primer for the construction of SOD1-disrupting
      plasmid)

<400> 12
ggtacctccg atgataggaa tgtgag                                       26


<210> 13
<211> 26
<212> DNA
<213> Artificial Sequence

<220>
<223> Description of Artificial Sequence:Sod15
      (antisense primer for the construction of
      SOD1-disrupting plasmid)

<400> 13
gaattcagtt caacggagga ggacac                                       26


<210> 14
<211> 26

<212> DNA
<213> Artificial Sequence

<220>
<223> Description of Artificial Sequence:Sod47 (sense
      primer for the construction of SOD2-disrupting
      plasmid)

<400> 14
gaattcggag gaggacacat caaccg                                          26


<210> 15
<211> 26
<212> DNA
<213> Artificial Sequence

<220>
<223> Description of Artificial Sequence:Sod48
      (antisense primer for the construction of
      SOD2-disrupting plasmid)

<400> 15
ggtacctgta ctggaggtag aaagcg                                          26


<210> 16
<211> 23
<212> DNA
<213> Artificial Sequence

<220>
<223> Description of Artificial Sequence:Sod2 (sense
      primer for cloning of CAT gene)

<400> 16
mgnttytcna cngtnggngg nga                                             23


<210> 17
<211> 23
<212> DNA
<213> Artificial Sequence

<220>
<223> Description of Artificial Sequence:Cat5 (antisense
      primer for cloning of CAT gene)

<400> 17
ckrtgnckyt gngtrtcngg rta                                             23

## Claims

1. A process for producing carotenoids which comprises cultivating a recombinant organism whose gene for one or more active oxygen species-quenching factor(s) is substantially disrupted with the aid of a disruption cassette specific to the said gene, and recovering carotenoids from the culture.

2. A process according to claim 1, wherein the host organism of the said recombinant organism belongs to the kingdom of *Monera, Protista or Fungi.*

3. A process according to claim 1 or 2, wherein the host organism of the said recombinant organism belongs to the genus *Erwinia*, *Rhodobacter, Myxococcus, Flavobacter, Paracoccus, Synechococcus, Synechocystis, Agrobacterium, Streptomyces, Haematococcus, Dunaliella, Phaffia, Xanthophyllomyces, Neurospora*, *Rhodotorula, Blakeslea,* or *Phycomyces.*

4. A process according to claim 3, wherein the host organism of the said recombinant organism is a strain of *P. rhodozyma*

5. A process according to any one of claims 1 - 4, wherein the active oxygen species-quenching factor(s) is(are) mitochondrial superoxide dismutase (SOD), cytoplasmic superoxide dismutase (SOD) and/or catalase.

6. A recombinant organism producible of carotenoids, which is characterized in that the gene for at least one active oxygen species-quenching factor is substantially disrupted with the aid of introduction of a disruption cassette specific to the said gene.

7. A recombinant organism according to claim 6, wherein the host organism of the said recombinant organism belongs to the kingdom of *Monera, Protista or Fungi,* more preferably to the genus *Erwinia, Rhodobacter, Myxococcus, Flavobacter*, *Paracoccus, Synechococcus, Synechocystis, Agrobacterium, Streptomyces, Haematococcus, Dunaliella, Phaffia, Xanthophyllomyces, Neurospora*, *Rhodotorula, Blakeslea,* or *Phycomyces.*

8. A recombinant organism according to claim 6 or 7, wherein the active oxygen species-quenching factor(s) to be disrupted is(are) mitochondrial superoxide dismutase (SOD), cytoplasmic superoxide dismutase (SOD) and/or catalase.

9. A disruption cassette for disrupting a gene coding for an active oxygen species-quenching factor effective on carotenogenesis in an organism which is producible of carotenoids, which comprises a partial nucleotide sequence substantially identical to a part of the DNA sequence coding for an active oxygen species-quenching factor and a selectable marker gene.

10. A disruption cassette according to claim 9, wherein the said organism belongs to the kingdom of *Monern, Protista or Fungi,* more preferably to the genus *Erwinia, Rhodobacter, Myxococcus*, *Flavobacter, Paracoccus, Synechococcus, Synechocystis, Agrobacterium, Streptomyces, Haematococcus, Dunaliella, Phaffia, Xanthophyllomyces, Neurospora, Rhodotorula, Blakeslea,* or *Phycomyces.*

11. A disruption cassette according to claim 9 or 10, wherein the active oxygen species-quenching factor(s) to be disrupted is(are) mitochondrial superoxide dismutase (SOD), cytoplasmic superoxide dismutase (SOD) and/or catalase.

12. A disruption cassette according to any one of claims 9 to 11, wherein the partial nucleotide sequence coding for an active oxygen species-quenching factor is identical to at least a part of the DNA sequence coding for the active oxygen species-quenching factor of the organism into which the disruption cassette is to be introduced.

13. A disruption cassette according to claim 12, wherein the partial nucleotide sequence substantially identical to a part of the DNA sequence coding for an active oxygen species-quenching factor comprises at least one deletion and/or mutation compared to the corresponding functional gene.

14. A recombinant DNA sequence coding for an active oxygen species-quenching factor effective on carotenogenesis in an organism producible of carotenoids.

15. A recombinant DNA sequence according to claim 14, wherein the said organism belongs to the kingdom of *Monera, Protista or Fungi.*

16. A recombinant DNA sequence according to claim 14 or 15, wherein the said organism belongs to the kingdom of *Monera, Protista or Fungi,* more preferably to the genus *Erwinia, Rhodobacter, Myxococcus, Flavobacter, Paracoccus, Synechococcus, Synechocystis, Agrobacterium, Streptomyces, Haematococcus, Dunaliella, Phaffia, Xanthophyllomyces Neurospora, Rhodotorula, Blakeslea, or Phycomyces.*

17. A recombinant DNA sequence according to any one of claims 14 - 16, which is a sequence originated from a microorganism of *P. rhodozyma.*

18. A recombinant DNA sequence according to any one of claims 14 - 17, wherein the active oxygen species-quenching factor is mitochondrial superoxide dismutase.

19. A recombinant DNA sequence according to claim 18, wherein the sequence is identified by SEQ ID NO: 1 or 4, or has high homology to the sequence of SEQ ID NO: 1 or 4 enough to recombine with either of the sequences SEQ ID NOs: 1 and 4.

20. A recombinant DNA sequence according to claim 14 - 17, wherein the active oxygen species-quenching factor is cytoplasmic superoxide dismutase.

21. A recombinant DNA sequence according to claim 20, wherein the sequence is identified by SEQ ID NO: 2 or 6, or has high homology to the sequence of SEQ ID NO: 2 or 6 enough to recombine with either of the sequences of SEQ ID NOs: 2 and 6.

22. A recombinant DNA sequence according to claim 14 - 17, wherein the active oxygen species-quenching factor is catalase.

23. A recombinant DNA sequence according to claim 22, wherein the sequence is identified by SEQ ID NO: 3 or 8, or has high homology to the sequence of SEQ ID NO: 3 or 8 enough to recombine with either of the sequences of SEQ ID NOs: 3 and 8.

24. A recombinant DNA fragment which comprises a coding region for a transit peptide upstream the coding region of an objective protein.

25. A recombinant DNA fragment according to claim 24, wherein the objective protein is mitochondrial superoxide dismutase.

26. A method for locating an objective protein in mitochondria which comprises expressing the recombinant DNA fragment defined in claim 24 or 25 in an appropriate recombinant host organism.

27. A polynucleotide for use as probe or primer for cloning gene for the active oxygen species-quenching factor(s) effective on carotenogenesis in an organism producible of carotenoids.

28. A polynucleotide according to claim 27, wherein the active oxygen species-quenching factor(s) is(are) mitochondrial superoxide dismutase (SOD), cytoplasmic superoxide dismutase (SOD) and/or catalase.
